(19) Europäisches Patentamt
European Patent Office
Office européen des brevets

(11) Veröffentlichungsnummer: **0 167 008**
**B1**

(12) # EUROPÄISCHE PATENTSCHRIFT

(45) Veröffentlichungstag der Patentschrift: **06.09.89**

(51) Int. Cl.⁴: **C 07 D 493/04**, C 07 D 519/00, A 61 K 31/34, A 61 K 31/52 // (C07D493/04, 307:00, 307:00), (C07D519/00, 493:00, 473:00)

(21) Anmeldenummer: **85106993.0**

(22) Anmeldetag: **05.06.85**

(54) **1,4:3,6-Dianhydro-hexit-Derivate, Verfahren zu ihrer Herstellung und ihre Verwendung als Arzneimittel.**

(30) Priorität: **06.06.84 DE 3421072**

(43) Veröffentlichungstag der Anmeldung: **08.01.86 Patentblatt 86/2**

(45) Bekanntmachung des Hinweises auf die Patenterteilung: **06.09.89 Patentblatt 89/36**

(84) Benannte Vertragsstaaten: **AT BE CH DE FR GB IT LI LU NL**

(56) Entgegenhaltungen:
**EP-A- 0 044 928**
**FR-M- 4 061**
**GB-A- 1 356 374**

(73) Patentinhaber: **Heinrich Mack Nachf., Postfach 140, D-7918 Illertissen (DE)**

(72) Erfinder: **Stoss, Peter, Dr. Dipl.-Chem., Mozartstrasse 15, D-7918 Illertissen (DE)**
Erfinder: **Leitold, Matyas, Dr., Klauflügelweg 21, D-7950 Biberach (DE)**

(74) Vertreter: **Lederer, Franz, Dr., Van der Werth, Lederer & Riederer Patentanwälte Lucile-Grahn-Strasse 22, D-8000 München 80 (DE)**

EP 0 167 008 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Verbindungsklasse der 1,4 : 3,6-Dianhydrohexite ist seit 100 Jahren bekannt. Als ersten Vertreter hat A. Fauconnier, Bull. Soc. Chim. France, 41, 119 (1884), Isomannid beschrieben. Diesem kommt, wie auch dem später bekannt gewordenen Isosorbid, eine diuretische Wirkung zu; siehe z. B. Proc. Soc. exp. Biol. Med. 119, 39 (1965) sowie USP 2 143 324. Inzwischen sind eine Reihe von Derivaten als pharmakologisch wirksam erkannt worden. Von diesen befinden sich das Isosorbid-2,5-dinitrat seit vielen Jahren, das Isosorbid-5-mononitrat seit kurzem als Koronartherapeutika auf dem Markt.

Zum Stand der Technik gehören ferner folgende Druckschriften: Die DE-OS-2 221 080 hat Mononitratester des Isosorbids zum Inhalt, die zur Behandlung von Angina pectoris eingesetzt werden können. In der GB-PS-1 027 891 sind Nicotinsäureester von 1,4 : 3,6-Dianhydrohexiten mit vasodilatatorischen Eigenschaften beschrieben. Muskelrelaxierend wirkende Isohexid-ether werden unter anderem in der USP-4 169 152 aufgeführt. O-substituierte 1,4 : 3,6-Dianhydro-hexit-mononitrate mit Wirkung auf das Herz- und Kreislaufsystem sind Gegenstand der DE-OS-3 028 289. Die GB-A-1 356 374 offenbart O-substituierte Mononitratester von Isosorbid und ihre Brauchbarkeit als koronarwirksame Arzneimittel.

Neben diesen Isohexid-Derivaten sind verschiedene Desoxy-1,4 : 3,6-dianhydro-hexite mit pharmakologischer Wirkung bekannt, bei denen anstelle des Sauerstoffs in 2- bzw. 5-Stellung eine gegebenenfalls substituierte Aminogruppe steht: J. Indian Chem. 16B, 153 (1978), DE-OS 3 028 272, DE-OS 3 028 273, DE-OS 3 028 288 und DE-OS 3 028 340. Schließlich sind auch Verbindungen hergestellt worden, die keine freie oder substituierte Hydroxylgruppe mehr enthalten, nämlich gemäß DE-OS 3 109 532, die 1,4 : 3,6-Dianhydro-2,5-diazido-2,5-dideoxy-hexite mit hypnotischer Wirkung zum Inhalt hat [siehe auch Carbohydrate Res. 85, 259 (1980)].

Die erfindungsgemäßen 1,4 : 3,6-Dianhydrohexit-Derivate unterscheiden sich strukturell von allen diesen vorbeschriebenen Verbindungen dadurch, daß sie als charakteristisches Strukturelement eine N-substituierte 3-Amino-2-hydroxypropyl-Gruppe, die etherartig mit dem Grundkörper der Dianhydro-zuckeralkohole verknüpft ist, enthalten.

Bekanntlich stellt eine N-alkylierte-propanolamin-Funktion die pharmakophore Wirkgruppe einer großen Anzahl von sogenannten β-Rezeptorenblockern dar. Die entsprechende Stoffklasse ist unter dem Oberbegriff «Aryloxypropanolamine» zusammengefaßt. Durch die Bezeichnung «Aryl» wird zum Ausdruck gebracht, was auch nach der herrschenden Lehrmeinung als unverzichtbar galt, daß nämlich für das Zustandekommen einer β-antagonistischen Wirkung die Gegenwart eines aromatischen bzw. heteroaromatischen Grundkörpers zwingend erforderlich ist. (Siehe z. B. E. Schröder, C. Rufer, R. Schmiechen, Pharmazeutische Chemie, Thieme-Verlag 1982, S. 682 ff.) Diesem Erfordernis entsprechen auch sämtliche bisher therapeutisch eingesetzten oder in klinischer Erprobung befindlichen Arzneimittel dieses Strukturtyps. So offenbart die FR-M-4061 Propanolaminderivate von Phenol, die als beta-Rezeptorblocker brauchbar sind.

Zwar sind bereits vereinzelte Versuche bekannt geworden, dieses Prinzip zu durchbrechen. Jedoch war diesen Versuchen bisher kein nutzbarer Erfolg beschieden. So werden aliphatische Amino-hydroxy-propandiol-ether in J. Med. Chem. 23, 620 (1980) sowie in Arch. Pharm. 312, 857 (1979), Arch. Pharm. 312, 881 (1979) und Arch. Pharm. 317, 63 (1984) beschrieben. Weitere Vertreter dieses Strukturtyps sind Gegenstand der Druckschriften DE-OS 2 558 285, EP 37 777 und EP 87 378.

Keine dieser vorbeschriebenen 3-Amino-propandiol-Verbindungen enthält jedoch einen 1,4 : 3,6-Dianhydro-hexit-Rest.

Erfindungsgemäß werden neuartig strukturierte Verbindungen zur Verfügung gestellt, mit denen sich die Möglichkeit eröffnet, sowohl die therapeutische Einsatzbreite von Isohexid-Derivaten auszuweiten, als auch die Klasse der β-Rezeptorenblocker durch bisher nicht bekannte Strukturvarianten mit überlegenen Eigenschaften zu bereichern. Überraschenderweise zeigen die erfindungsgemäßen Verbindungen ein pharmakologisches Wirkprofil, wie es weder von den bisher bekannten 1,4: 3,6-Dianhydro-hexiten noch von Aryloxypropanolaminen abgedeckt wird. Diese vorteilhaften pharmakologischen Eigenschaften stellen deshalb eine nicht vorhersehbare Erweiterung der therapeutischen Möglichkeiten und damit eine Bereicherung der Pharmazie dar.

Propanolamine der angesprochenen Struktur existieren bekanntlich als Racemate, von denen in der Regel nur ein Antipode die gewünschte pharmakologische Wirkung entfaltet. Nach den üblichen Synthesewegen erhält man jedoch stets racemische Verbindungen. Das ist auch der Grund dafür, daß sämtliche bekannten β-Blocker, von einer Ausnahme (Timolol) abgesehen, therapeutisch als Racemate zum Einsatz kommen. Man zieht hier also 50% Verunreinigung mit dem «falschen» Enantiomer einer mühevollen Racematspaltung vor. Zur Herstellung des erwünschten Enantiomeren aus dem Racemat sind jedem Fachmann geläufige, aufwendige Verfahren erforderlich. Eines dieser Verfahren besteht z. B. aus der Überführung in ein Salz oder Derivat mit einem optisch aktiven Partner, Trennung des so erzeugten diastereomeren Salzes oder Derivates durch fraktionierte Kristallisation, Destillation oder auf andere geeignete Weise und nachfolgende Spaltung von Salz bzw. Derivat in den optisch reinen Grundkörper. Demgegenüber bilden die erfindungsgemäßen Verbindungen durch die intramolekulare Kombination eines 1,4 : 3,6-Dianhydrohexit-Restes mit einer Propanolamin-Gruppe von vornherein Diastereoisomere, die, falls gewünscht, direkt, d. h. ohne zusätzliche chemische Reaktionen, trennbar sind.

Gegenstand der vorliegenden Erfindung sind daher neue 1,4 : 3,6-Dianhydro-hexit-Derivate der allgemeinen Formel I

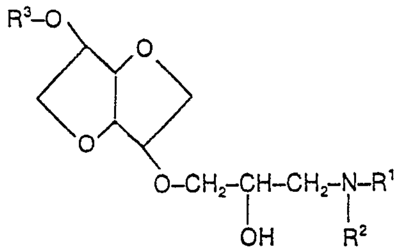

I

worin

$R^1$ Wasserstoff oder eine Benzylgruppe bedeutet,

$R^2$ einen geradkettigen oder verzweigten Alkylrest mit 1–4 Kohlenstoffatomen oder einen ω-Theophyllin-7-yl-alkyl-Rest, wobei dieser Alkylrest 2–3 Kohlenstoffatome aufweisen kann, bedeutet

oder worin

$R^1$ und $R^2$ gemeinsam mit dem Stickstoffatom, an das sie gebunden sind, den Theophyllin-7-yl-Rest, den Theobromin-1-yl-Rest, den an der 6-Aminogruppe gegebenenfalls mono- oder disubstituierten Aden-9-yl-Rest, den an der 2-Aminogruppe gegebenenfalls substituierten Guan-9-yl-Rest, den 6-Chlorpurin-9-yl-Rest oder den 6-Alkylmercaptopurin-9-yl-Rest oder den an der Aminogruppe gegebenenfalls substituierten 2-Amino-6-chlor-purin-9-yl-Rest darstellt

und worin

$R^3$ Wasserstoff, einen geradkettigen oder verzweigten, gesättigten oder ungesättigten Acylrest mit 1–20 Kohlenstoffatomen, einen Pyridylcarbonylrest, eine $NO_2$-Gruppe oder einen Rest der Formel

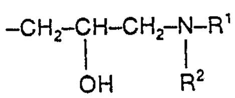

darstellt, worin $R^1$ und $R^2$ die oben angegebene Bedeutung besitzen

sowie deren Salze mit anorganischen und organischen Säuren, vorzugsweise deren physiologisch verträgliche Salze.

Für den Fall, daß $R^1$ und $R^2$ gemeinsam mit dem Stickstoffatom, an das sie gebunden sind, einen an der Aminogruppe substituierten heterocyclischen Rest bedeuten, können die Substituenten niedere aliphatische und aromatische Acylreste, wie z. B. Acetyl-, Propionyl-, Butyryl-, Isobutyryl- sowie Benzoyl- und Toluylreste sein.

1,4 : 3,6-Dianhydro-hexite sind bicyclische Verbindungen, die in Form zweier cis-verknüpfter Tetrahydrofuranringe vorliegen, von denen jeder eine Hydroxygruppe trägt. Ihre Herstellung erfolgt durch Dehydratisierung der Hexite, das sind sechswertige, geradkettige, gesättigte, aliphatische Alkohole mit 6 Kohlenstoffatomen. Neben der Bezeichnung 1,4 : 3,6-Dianhydro-hexite sind noch die Namen 1,4 : 3,6-Dianhydro-hexitole, Isohexide und Isohexitole im Gebrauch. Nach der systematischen Nomenklatur werden die Verbindungen als Brückenringsysteme mit der Bezeichnung 2,6-Dioxabicyclo[3.3.0]octan-4,8-diole, als anellierte Systeme mit der Bezeichnung Hexahydro-furo[3.2.-b]furan-3,6-diole belegt.

Die Grundkörper der hier behandelten Derivate bestehen aus einem der nachfolgend aufgeführten, stereoisomeren, unter Epimerisierung ineinander überführbaren 1,4 : 3,6-Dianhydro-hexite, nämlich

1,4 : 3,6-Dianhydro-L-idit (Isoidid) folgender Struktur,

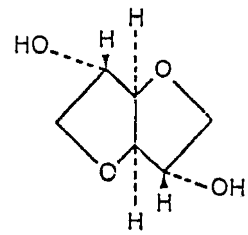

bei dem die OH-Gruppen in 2- und 5-Stellung jeweils exo-Konfiguration aufweisen

oder

1,4 : 3,6-Dianhydro-D-glucit (1,4 : 3,6-Dianhydro-D-sorbit, Isosorbid) folgender Struktur,

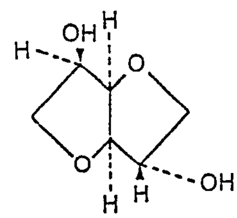

der eine 2-exo-ständige sowie eine 5-endo-ständige OH-Gruppe aufweist und dessen O-Derivate, bei nichtidentischen Substituenten, in zwei isomeren Formen auftreten

oder

1,4 : 3,6-Dianhydro-D-mannit (Isomannid) folgender Struktur,

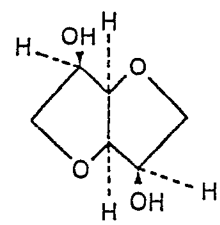

der zwei endo-ständige OH-Gruppen aufweist.

Im Gegensatz zu den Glucit-Derivaten ist bei den Idit- und Mannit-Derivaten eine Unterscheidung zwischen einer Substitution in der 2- und 5-Position nicht möglich. Eine kurze Zusammenfassung über die Stereochemie der 1,4 : 3,6-Dianhydro-hexite geben J. A. Mills in Advances in Carbohydrate Chemistry 10, 1–53 (1955) sowie L. Hough und A. C. Richardson in Rodd's Chemistry of Carbon Compounds, 2. Ed. Vol 1, F, Elsevier, 1967, S. 51–55.

1,4 : 3,6-Dianhydro-hexite besitzen vier chirale Zentren, nämlich die Kohlenstoffatome 2, 3, 4 und 5. Die zur Herstellung der erfindungsgemäßen Verbindungen eingesetzten Ausgangsprodukte liegen in optisch reiner Form, als L-Isoidid, D-Isosorbid und D-Isomannid vor. Sie sind aus den entsprechenden natürlich vorkommenden, optisch aktiven Zuckeralkoholen zugänglich. Die in den erfindungsgemäßen Verbindungen enthaltene N-substituierte 3-Amino-2-propanol-Seitenkette

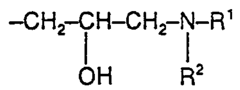

besitzt im C-2 ebenfalls ein asymmetrisches Kohlenstoffatom. Aus diesem Grund treten die erfindungsgemäßen Verbindungen der allgemeinen Formel I in diastereoisomeren Formen auf. Sowohl die Diastereoisomerengemische, als auch die getrennten, konfigurativ einheitlichen Komponenten sind Gegenstand der vorliegenden Erfindung.

Gegenstand der vorliegenden Erfindung sind demnach

1,4 : 3,6-Dianhydro-L-idit-Derivate der allgemeinen Formel II

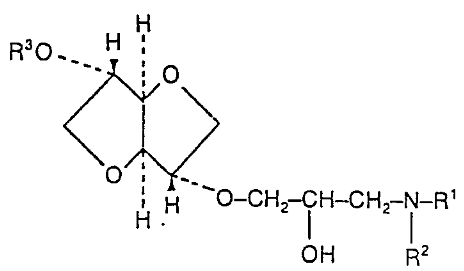

II

worin $R^1$, $R^2$ und $R^3$ die oben angegebene Bedeutung besitzen, sowie deren Salze,

1,4 : 3,6-Dianhydro-D-glucit-Derivate der allgemeinen Formeln III und IV,

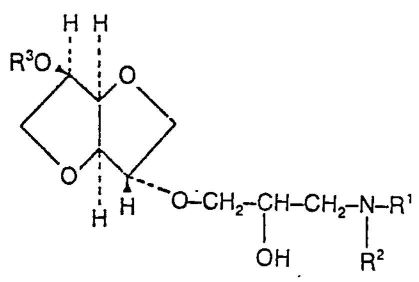

III

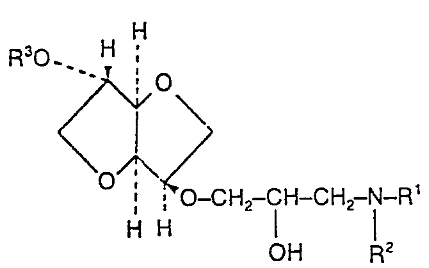

IV

worin $R^1$, $R^2$ und $R^3$ die oben angegebene Bedeutung besitzen, sowie deren Salze,

1,4 : 3,6-Dianhydro-D-mannit-Derivate der allgemeinen Formel V,

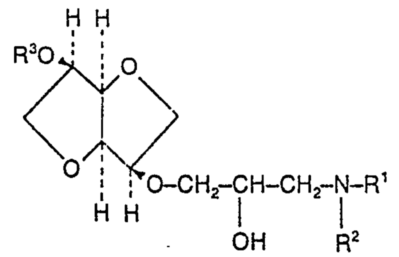

V

worin $R^1$, $R^2$ und $R^3$ die oben angegebene Bedeutung besitzen, sowie deren Salze.

Die Verbindungen der vorliegenden Erfindung können in an sich bekannter Weise hergestellt werden. Eines der Verfahren zur Herstellung von Verbindungen der allgemeinen Formel I ist dadurch gekennzeichnet, daß man einen (2,3-Epoxypropyl)-1,4 : 3,6-dianhydro-hexit der allgemeinen Formel VI,

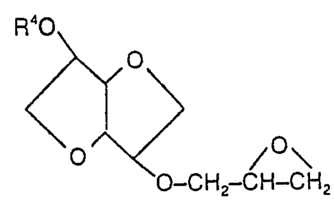

VI

worin $R^4$ Wasserstoff, einen geradkettigen oder verzweigten, gesättigten oder ungesättigten Acylrest mit 1–20 Kohlenstoffatomen, einen Pyridylcarbonylrest, eine $NO_2$-Gruppe oder einen 2,3-Epoxypropylrest der Formel

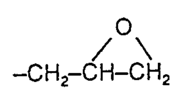

darstellt, in an sich bekannter Weise mit einer Verbindung der allgemeinen Formel VII,

$$HNR^1R^2 \qquad VII$$

worin $R^1$ und $R^2$ die oben angegebene Bedeutung besitzen, umsetzt und das Reaktionsprodukt der allgemeinen Formel I gegebenenfalls in ein Salz mit einer anorganischen oder organischen Säure, vorzugsweise in ein pharmazeutisch akzeptables Salz, überführt.

Wird bei dieser Umsetzung ein Diastereoisomerengemisch als Edukt verwendet, so ist das Produkt ebenfalls ein Diastereoisomerengemisch. Setzt man ein optisch einheitliches Isomer als Reaktionspartner ein, so erhält man jeweils ein reines Diastereoisomer. Diastereoisomerengemische können bei Bedarf durch bekannte Trenntechniken in die reinen Komponenten zerlegt werden.

Die Umsetzung von Epoxiden der allgemeinen Formel VI mit Verbindungen der allgemeinen Formel VII kann sowohl ohne Lösungsmittel, als auch unter Zusatz eines geeigneten, unter den Reaktionsbedingungen inerten Lösungsmittels erfolgen. Als solche kommen beispielsweise in Frage: Wasser; niedere Alkohole, wie Methanol, Ethanol, 1-Propanol, 2-Propanol und Butanol; Ether, wie Diethylether, Diisopropylether, Dibutylether, Ethylenglykol-mono und -dimethyl und -ethylether, Tetrahydrofuran und Dioxan; DMF sowie DMSO. Die Reaktionstemperatur kann zwischen 0 °C und 200 °C liegen, vorwiegend im Bereich von Raumtemperatur bis zum Siedepunkt der Verbindung VII oder des gegebenenfalls eingesetzten Lösungsmittels. Die Molverhältnisse der Reaktionspartner VI und VII können nach Wunsch gewählt werden und vom Verhältnis 1 : 1 bis 1 : 100 schwanken. Die Reaktionszeiten können im Bereich von 0,5 bis 24 Stunden liegen.

Nach beendeter Umsetzung werden die Reaktionsprodukte der allgemeinen Formel I auf übliche Weise isoliert, gereinigt und gegebenenfalls in das Salz einer anorganischen oder organischen Säure, vorzugsweise in ein pharmazeutisch akzeptables Salz überführt. Beispiele für derartige Salze sind Hydrochloride, Hydrobromide, Nitrate, Sulfate, Phosphate, Acetate, Oxalate, Maleate, Fumarate, Tartrate, Lactate, Maleinate, Malonate, Citrate, Salicylate, Methansulfonate, Benzolsulfonate, p-Toluolsulfonate und Naphthalinsulfonate.

Diese oder andere Salze der neuen Verbindungen, wie z. B. Pikrate, können auch zur Reinigung der erhaltenen freien Basen dienen, indem man die freie Base in ein Salz überführt, dieses abtrennt und gegebenenfalls umkristallisiert oder anderweitig reinigt und aus dem Salz wiederum die Base freisetzt.

Verbindungen der allgemeinen Formel VI, worin $R^4$ die angegebene Bedeutung besitzt, sind in an sich bekannter Weise erhältlich aus 1,4 : 3,6-Dianhydro-hexiten der allgemeinen Formel VIII,

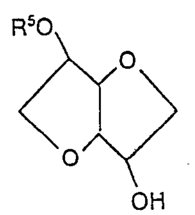

VIII

worin $R^5$ Wasserstoff, einen geradkettigen oder verzweigten, gesättigten oder ungesättigten Acylrest mit 1–20 Kohlenstoffatomen, einen Pyridylcarbonylrest oder eine $NO_2$-Gruppe darstellt

a) durch Umsetzung mit einem Epihalohydrin der Formel IX,

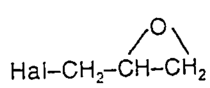

IX

worin Hal für einen Halogenrest, insbesondere für einen Chlor-, Brom- oder Jod-Rest steht, in Gegenwart einer Base

oder

b) durch Umsetzung mit einem Allylhalogenid der Formel X,

$$Hal{-}CH_2{-}CH=CH_2 \qquad X$$

worin Hal für einen Halogenrest, insbesondere für einen Chlor-, Brom- oder Jod-Rest steht, in Gegenwart einer Base, wobei zunächst Verbindungen der allgemeinen Formel XI gebildet werden,

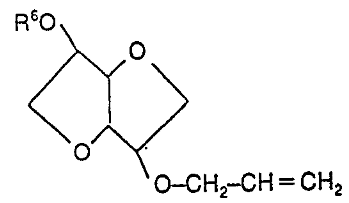

XI

worin $R^6$ Wasserstoff, einen geradkettigen oder verzweigten, gesättigten oder ungesättigten Acylrest mit 1–20 Kohlenstoffatomen, einen Pyridylcarbonylrest, eine $NO_2$-Gruppe oder einen Allyl-Rest der Formel

$$-CH_2{-}CH=CH_2$$

darstellt und nachfolgende Oxydation der Verbindungen XI zu Verbindungen der allgemeinen Formel VI.

Für die Epoxydierung der Verbindungen XI können alle dem Fachmann geläufigen Oxydationsmittel eingesetzt werden, wie z. B. Peressigsäure, Perbenzoesäure, m-Chlor-perbenzoesäure, Monoperphthalsäure und andere. Zur Methodik und den Reaktionsbedingungen siehe z. B. Houben-Weyl VI/3, S. 385 ff.

Beim Einsatz eines racemischen Epihalohydrins der allgemeinen Formel IX als Reaktionspartner nach a) ist das erhaltene Produkt ein Diastereoisomerengemisch. Dieses kann bei Bedarf durch herkömmliche Trenntechniken in die Komponenten zerlegt werden. Setzt man ein enantiomerenreines Epihalohydrin in die obige Reaktion ein, so erhält man, in Abhängigkeit von der absoluten Konfiguration des verwendeten Enantiomers, jeweils eines der möglichen Diastereoisomere.

Bei der nach b) erforderlichen Epoxydation der Allylverbindungen XI wird ein neues Chiralitätszentrum geschaffen. Deshalb liegen die auf diesem Weg erhaltenen 2,3-Epoxypropyl-Derivate als Diastereoisomerengemische vor. Diese können bei Bedarf durch bekannte Trennmethoden in die reinen Isomeren gespalten werden.

Die Umsetzungen von VIII mit IX oder mit X können sowohl ohne Lösungsmittel, als auch unter Zusatz eines geeigneten, unter den Reaktionsbedingungen inerten Lösungsmittels erfolgen. Als solche kommen beispielsweise in Frage: Aceton, Ethyl-methyl-keton, Benzol, Toluol, Xylol, Ethylenglykol-dimethyl und -diethylether, Dioxan, Tetrahydrofuran, DMF und DMSO.

Als Basen für diese Reaktionen können beispielsweise eingesetzt werden: Natriumhydrid, Kaliumhydrid, Natriumhydroxid, Kaliumhydroxid, Natriumcarbonat, Kaliumcarbonat oder Kalium-tert.-butylat. Die Reaktionstemperaturen können zwischen 0 °C und 180 °C liegen, vorwiegend im

Bereich von Raumtemperatur bis zum Siedepunkt des eingesetzten Epihalohydrins, Allylhalogenids oder Lösungsmittels. Die Molverhältnisse der Reaktionspartner VIII und IX bzw. X können nach Wunsch gewählt werden und vom Verhältnis 1 : 1 bis 1 : 100 schwanken. Die Reaktionszeiten liegen im Bereich von 0,5 bis 24 Stunden.

Setzt man als Ausgangsprodukte Verbindungen der allgemeinen Formel VIII ein, in der $R^5$ Wasserstoff darstellt, so ist bei den Umsetzungen nach a) und b) sowohl die Bildung von mono- als auch von di-substituierten Produkten möglich. Im Falle des 1,4 : 3,6-Dianhydro-D-glucits (Isosorbid) sind darüber hinaus zwei isomere Monosubstitutionsprodukte zu erwarten. Durch geeignete Wahl der Molverhältnisse und der Reaktionsbedingungen gelingt es, die Umsetzung bevorzugt in die jeweils gewünschte Richtung zu lenken. Außerdem können Gemische von Mono- und Disubstitutionsprodukten, sowie der isomeren 2- und 5-substituierten Isosorbide mit Hilfe von Methoden getrennt werden, die jedem Fachmann geläufig sind, wie z. B. Kristallisation, Destillation, Extraktion oder chromatografische Verfahren.

Verbindungen der allgemeinen Formel VI, worin $R^4$, mit Ausnahme von Wasserstoff und dem 2,3-Epoxypropylrest, alle oben angeführten Bedeutungen besitzt, sind auch erhältlich aus Verbindungen der allgemeinen Formel VI, worin $R^4$ Wasserstoff bedeutet, durch Umsetzung mit geradkettigen oder verzweigten, gesättigten oder ungesättigten Acylhalogeniden mit 1–20 Kohlenstoffatomen oder den entsprechenden Carbonsäureanhydriden, mit Pyridylcarbonylhalogeniden und mit Salpetersäure. Diese Umsetzungen erfolgen auf an sich bekannte Weise nach Methoden, die dem Fachmann geläufig sind.

Auf gleiche Weise kann man Verbindungen der allgemeinen Formel XI, worin $R^6$, mit Ausnahme von Wasserstoff und einem Allyl-Rest, alle angegebenen Bedeutungen besitzt, herstellen, indem man Verbindungen der allgemeinen Formel XI, worin $R^6$ Wasserstoff bedeutet, mit geradkettigen oder verzweigten, gesättigten oder ungesättigten Acylhalogeniden mit 1–20 Kohlenstoffatomen oder den entsprechenden Carbonsäureanhydriden, mit Pyridylcarbonylchloriden und mit Salpetersäure umsetzt. Auch diese Reaktionen erfolgen auf an sich bekannte Weise und nach den üblichen Methoden.

2,5-Bis-(2,3-epoxypropyl)-ether des Isosorbids, Isomannids und Isoidids sind in dem US-Patent 3 272 845 beschrieben. Mono-epoxypropyl-1,4 : 3,6-dianhydro-hexite und sämtliche Derivate hiervon sind bisher noch nicht bekannt und stellen somit neue Zwischenprodukte dar.

Beispielhaft für Verbindungen der allgemeinen Formel VI, die in der vorliegenden Erfindung als neue Zwischenprodukte auftreten können, werden aufgeführt:

2-(2,3-Epoxypropyl)-isosorbid
5-(2,3-Epoxypropyl)-isosorbid
2-(2,4-Epoxypropyl)-isomannid
2-(2,4-Epoxypropyl)-isoidid
2-(2,3-Epoxypropyl)-isosorbid-5-nitrat
5-(2,3-Epoxypropyl)-isosorbid-2-nitrat
2-(2,3-Epoxypropyl)-isomannid-5-nitrat
2-(2,3-Epoxypropyl)-isoidid-5-nitrat
2-(2,3-Epoxypropyl)-isosorbid-5-nicotinat
5-(2,4-Epoxypropyl)-isosorbid-2-nicotinat
2-(2,3-Epoxypropyl)-isosorbid-5-isonicotinat
5-(2,3-Epoxypropyl)-isosorbid-2-isonicotinat
2-(2,3-Epoxypropyl)-isomannid-5-nicotinat
2-(2,3-Epoxypropyl)-isomannid-5-isonicotinat
2-(2,3-Epoxypropyl)-isoidid-5-nicotinat
2-(2,3-Epoxypropyl)-isoidid-5-isonicotinat
2-(2,3-Epoxypropyl)-isosorbid-5-acylat
5-(2,3-Epoxypropyl)-isosorbid-2-acylat
2-(2,3-Epoxypropyl)-isomannid-5-acylat
2-(2,3-Epoxypropyl)-isoidid-5-acylat

Bei den vier letztgenannten Verbindungen bezieht sich der Ausdruck «acylat» jeweils auf einen geradkettigen oder verzweigten, gesättigten oder ungesättigten Carbonsäurerest mit 1–20 Kohlenstoffatomen. Beispiele dafür sind Formiat, Acetat, Propionat, Acrylat, Butyrat, Isobutyrat, Pivaloat, Caprinat, Undecenoylat, Stearat, Arachinat.

Die Herstellung von 2,5-Diallyl-isosorbid und 2,5-Diallylisomannid ist beschrieben in J. Chem. Soc. 1947, 1405, sowie in J. Chem. Soc. 1950, 591. In der letzteren Arbeit sind zusätzlich 2-Allyl-isomannid, 2-Allyl-isomannid-5-acetat und die nicht aufgetrennten Gemische der beiden isomeren 2- und 5-Allyl-isosorbide und Allyl-isosorbid-acetate aufgeführt. Allylverbindungen des Isoidids, sowie sämtliche anderen in der vorliegenden Anmeldung eingesetzten Allylverbindungen der 1,4 : 3,6-Dianhydro-hexit-Derivate XI sind ebenfalls neu.

Als Beispiel für Verbindungen der allgemeinen Formel XI, die in der vorliegenden Erfindung als Zwischenprodukte auftreten können, seien genannt:

2-Allyl-isosorbid
5-Allyl-isosorbid
2-Allyl-isomannid
2-Allyl-isoidid
2-Allyl-isosorbid-5-nitrat
5-Allyl-isosorbid-2-nitrat
2-Allyl-isomannid-5-nitrat
2-Allyl-isoidid-5-nitrat
2-Allyl-isosorbid-5-nicotinat
5-Allyl-isosorbid-2-nicotinat
2-Allyl-isosorbid-5-isonicotinat
5-Allyl-isosorbid-2-isonicotinat
2-Allyl-isomannid-5-nicotinat
2-Allyl-isomannid-5-isonicotinat
2-Allyl-isoidid-5-nicotinat
2-Allyl-isoidid-5-isonicotinat
2-Allyl-isosorbid-5-acylat
5-Allyl-isosorbid-2-acylat
2-Allyl-isomannid-5-acylat
2-Allyl-isoidid-5-acylat

Bei den vier letztgenannten Verbindungen bezieht sich der Ausdruck «acylat» jeweils auf einen geradkettigen oder verzweigten, gesättigten oder ungesättigten Säurerest mit 1–20 Kohlenstoffatomen. Beispiele dafür sind Formiat, Acetat, Propionat, Acrylat, Butyrat, Isobutyrat, Pivaloat, Caprinat, Undecenoylat, Stearat, Arachinat.

Die erfindungsgemäßen Verbindungen zeichnen sich überraschenderweise durch ein breites pharmakologisches Wirkungsspektrum aus und stellen deshalb wertvolle Arzneistoffe dar. Sie besitzen β-sympatholytische, blutdrucksenkende, negativ inotrope und die Herzfrequenz senkende Wirksamkeit, zeigen spasmolytische, broncholytische und antitussive Eigenschaften und bewirken darüber hinaus eine Verbesserung der peripheren und zentralen Durchblutung. Sie können deshalb einerseits zur Prophylaxe und Behandlung von Herz- und Kreislauferkrankungen unterschiedlicher Genese, andererseits als Spasmolytika, Broncholytika, Antitussiva und auch als Stimulantien eingesetzt werden. Sie übertreffen damit alle bisher bekannten 1,4 : 3,6-Dianhydrohexit-Derivate und stellen somit eine Bereicherung der Pharmazie dar.

Definierte Beispiele für die erfindungsgemäße Verbindungsklasse sind:
2-(2-Hydroxy-3-isopropylamino-propyl)-isosorbid;
2-(2-Hydroxy-3-tert.-butylamino-propyl)-isosorbid;
2-(2-Hydroxy-3-tert.-butylamino-propyl)-isosorbid-5-nitrat;
5-(2-Hydroxy-3-tert.-butylamino-propyl)-isosorbid-2-nitrat;
5-(2-Hydroxy-3-tert.-butylamino-propyl)-isosorbid;
5-(2-Hydroxy-3-isopropylamino-propyl)-isosorbid;
2,5-Bis-(2-hydroxy-3-tert.-butylamino-propyl)-isosorbid;
2,5-Bis-(2-hydroxy-3-isopropylamino-propyl)-isosorbid;
2-(2-Hydroxy-3-isopropylamino-propyl)-isosorbid-5-nitrat;
2-(2-Hydroxy-3-isopropylamino-propyl)-isomannid;
2-(2-Hydroxy-3-isopropylamino-propyl)-isomannid-5-nitrat;
2,5-Bis-(2-hydroxy-3-isopropylamino-propyl)-isomannid;
2-(2-Hydroxy-3-isopropylamino-propyl)-isoidid;
2-(2-Hydroxy-3-isopropylamino-propyl)-isoidid-5-nitrat;
2-(2-Hydroxy-3-isopropylamino-propyl)-isosorbid-5-stearat;
2-(2-Hydroxy-3-isopropylamino-propyl)-isosorbid-5-isobutyrat;
2-(2-Hydroxy-3-isopropylamino-propyl)-isosorbid-5-pivaloat;
2-(2-Hydroxy-3-isopropylamino-propyl)-isosorbid-5-caprinat;
2-(2-Hydroxy-3-isopropylamino-propyl)-isosorbid-5-acetat;
2-(2-Hydroxy-3-isopropylamino-propyl)-isosorbid-5-undecylenat;
2-[2-Hydroxy-3-(7-theophyllinyl)-propyl]-isosorbid;
2-[2-Hydroxy-3-(7-theophyllinyl)-propyl]-isosorbid-5-nitrat;
5-[2-Hydroxy-3-(7-tehophyllinyl)-propyl]-isosorbid-2-nitrat;
Bis-2,5[2-hydroxy-3-(7-theophyllinyl)-propyl]-isosorbid;
2-[2-Hydroxy-3-(1-theobrominyl)-propyl]-isosorbid;
5-[3-[N-Benzyl-N-(2-(7-theophyllinyl)-ethylamino)]-2-hydroxy-propyl]-isosorbid;
2-[3-[N-Benzyl-N-(2-(7-theophyllinyl)-ethylamino)]-2-hydroxy-propyl]-isosorbid;
5-[3-[2-(7-Theophyllinyl)-ethylamino]-2-hydroxy-propyl]-isosorbid;
5-[3-[N-Benzyl-N-(2-(7-theophyllinyl)-ethylamino)]-2-hydroxy-propyl]-isosorbid-2-nitrat;
2-[3-[N-Benzyl-N-(3-(7-theophyllinyl)-propylamino)]-2-hydroxy-propyl]-isosorbid;
2-[3-[3-(7-Theophyllinyl)-propylamino]-2-hydroxy-propyl]-isosorbid;
2-[3-[2-(7-Theophyllinyl)-ethylamino]-2-hydroxy-propyl]-isosorbid;
2-[3-[N-Benzyl-N-(2-(7-theophyllinyl)-propylamino)]-2-hydroxy-propyl]-isosorbid-5-nitrat;
2-[3-[N-Benzyl-N-(3-(7-theophyllinyl)-propyl)-amino]-2-hydroxy-propyl]-isomannid;
2-[3-[3-(7-Theophyllinyl)-propylamino]-2-hydroxy-propyl]-isomannid;
5-[3-[2-(7-Theophyllinyl)-ethylamino]-2-hydroxy-propyl]-isosorbid-2-nitrat;
2-[3-[3-(7-Theophyllinyl)-propylamino]-2-hydroxy-propyl]-isosorbid-5-nitrat;
2-[3-($N^6$-Benzoyl-9-adenyl)-2-hydroxy-propyl]-isosorbid;
2-[3-(9-Adenyl)-2-hydroxy-propyl]-isosorbid;
Bis-2,5-[3-[N-benzyl-N-(2-(7-theophyllinyl)-ethyl)-amino]-2-hydroxy-propyl]-isomannid;
Bis-2,5-[3-[2-(7-theophyllinyl)-ethylamino]-2-hydroxy-propyl]-isomannid;
2-[3-(9-(6-Methylmercaptopurinyl))-2-hydroxy-propyl]-isosorbid.

Gegenstand der Erfindung ist auch die Verwendung von Verbindungen der allgemeinen Formel I und ihrer Salze als Arzneimittel. Derartige Arzneimittel können die erfindungsgemäßen Verbindungen oder deren Salze in einem Gehalt von 0,1 bis 99,9% enthalten. Die Dosierung kann wie gewünscht gewählt werden und im Bereich von 1 mg bis 500 mg liegen.

Als pharmazeutische Darreichungsformen kommen alle, dem Fachmann geläufigen Formulierungen in Frage, wie z. B. Suppositorien, Pulver, Granulate, Tabletten, Kapseln, Suspensionen, Liquida, Injectabilia und transdermale Systeme. Zur Herstellung pharmazeutischer Darreichungsformen können feste, halbfeste oder flüssige Trägermaterialien oder Verdünnungsmittel eingesetzt werden. Darin sind eingeschlossen Corrigentien, Bindemittel, Gleitmittel, Emulgatoren usw. Beispiele für derartige Mittel sind: Stärke, wie Kartoffel- und Getreidestärke, Zucker, wie Lactose, Sucrose, Glucose, Mannitol, Sorbitol, Cellulose, wie kristalline Cellulose, Methyl-cellulose, Calciumcarboxymethyl-cellulose, Natriumcarboxymethyl-cellulose und Hydroxypropylcellulose, anorganische Materialien wie Kaliumphosphat, Calciumsulfat, Calciumcarbonat und Talkum, Gelatine, Gummiarabicum, Polyvinylpyrrolidon, oberflächenaktive Substanzen wie Fettsäure-glyceride, Fettsäuresorbitan-ester, Fettsäureester von Sucrose und Polyglycerol und andere.

Einige Beispiele für Arzneimittelformulierungen, unter Benutzung der erfindungsgemäßen Verbindungen, sind nachfolgend aufgeführt:

Tabletten:

| Zusammensetzung | mg/Tablette |
|---|---|
| erfindungsgemäße Verbindung | 3 |
| Mikrokristalline Cellulose | 25 |
| Lactose | 17 |
| Carboxymethylcellulose-Calcium | 4,5 |
| Magnesiumstearat | 0,5 |

Obige Ingredienzien werden gesiebt, ausreichend und sorgfältig gemischt und auf einer geeigneten Tablettenpresse gepreßt.

Kapseln:

| Zusammensetzung | mg/Kapsel |
|---|---|
| erfindungsgemäße Verbindung | 10 |
| Lactose | 40 |
| Mikrokristalline Cellulose | 30 |
| Talcum | 10 |

Obige Ingredienzien werden gesiebt, ausreichend und sorgfältig gemischt und auf einer geeigneten Kapselfüllmaschine in Hartgelatinekapseln gefüllt.

Gegenstand der Erfindung ist auch die Verwendung der erfindungsgemäßen Verbindungen bei der Bekämpfung von Krankheiten, insbesondere bei der Behandlung von Herz- und Kreislauferkrankungen.

Die nachfolgenden Beispiele dienen zur Erläuterung der Erfindung. Sofern nicht anders vermerkt, handelt es sich bei den in den Beispielen aufgeführten Verbindungen jeweils um Diastereoisomerengemische.

Beispiel 1
2-(2-Hydroxy-3-isopropylamino-propyl)-isosorbid

a) 2-Allyl-isosorbid, 5-Allyl-isosorbid, 2,5-Diallyl-isosorbid:

450 g Isosorbid, 600 g Kaliumcarbonat und 400 ml Allylbromid werden in 1 l Toluol 24 Stunden unter Rückfluß erhitzt. Nach dem Erkalten filtriert man von den anorganischen Salzen ab, wäscht mit 0,5 l Toluol nach und engt das Filtrat ein. Der Rückstand wird in 1 l Wasser augenommen und 3mal mit je 200 ml Ether extrahiert. Die vereinigten Etherphasen werden mit 200 ml Wasser gegenextrahiert, getrocknet, eingeengt und i. Vak. über eine Kolonne destilliert. Man erhält 90 g 2,5-Diallyl-isosorbid, Sdp.$_{0,1}$: 84 °C.

Die vereinigten wäßrigen Phasen werden mit Natriumchlorid gesättigt und 2mal mit je 1 l Dichlormethan ausgeschüttelt. Nach dem Trocknen und Einengen der vereinigten organischen Phasen fraktioniert man den Rückstand i. Vak. über eine Kolonne. Man erhält

180 g 2-Allyl-isosorbid, Sdp.$_{0,1}$: 72 °C, Schmp. 35 °C aus Diethylether

20 g Mischfraktion, Sdp.$_{0,1}$: 72–112 °C

112 g 5-Allyl-isosorbid, Sdp.$_{0,1}$: 112 °C

b) 2-(2,3-Epoxypropyl)-isosorbid:

18,6 g 2-Allyl-isosorbid und 25 g 3-Chlorperbenzoesäure werden in 100 ml Chloroform 24 Stunden bei Raumtemperatur gerührt. Danach wird von der ausgefallenen 3-Chlor-benzoesäure abgesaugt und das Filtrat eingeengt. Der Rückstand wird in Wasser aufgenommen und mit Ether extrahiert. Der nach dem Einengen der Wasserphase verbleibende Rückstand kann entweder destilliert oder aus Ethylacetat/Ether umkristallisiert werden. Sdp.$_{0,1}$: 108 °C, Schmp. 74 °C, $[\alpha]_D^{20}$ + 53,9 (c = 0,892, Methanol).

Durch wiederholte Umkristallisation aus Ethylmethylketon erhält man ein Isomer vom Schmp. 88–90 °C, $[\alpha]_D^{20}$ + 66,1 (c = 1,022, Methanol).

Aus den Mutterlaugen kann das andere Isomer isoliert werden. Schmp. 60–62 ° (aus Ethylacetat/Ether), $[\alpha]_D^{20}$ + 40,4 (c = 0,904, Methanol).

c) 2-(2-Hydroxy-3-isopropylamino-propyl)-isosorbid:

20,2 g 2-(2,3-Epoxypropyl)-isosorbid (Isomerengemisch) werden mit 17,8 g Isopropylamin in 500 ml Ethanol 3 Stunden unter Rückfluß erhitzt. Danach entfernt man das Lösungsmittel und überschüssiges Amin am Rotationsverdampfer i. Vak. Der feste Rückstand wird aus Diisopropylether umkristallisiert. Ausbeute: 20,1 g (77%), Schmp. 65–67 °C, $[\alpha]_D^{20}$ + 38,5 (c = 0,80, Methanol).

Sowohl durch Trennung dieses Diastereoisomerengemisches, als auch ausgehend von den beiden isomerenreinen Epoxiden, sind die beiden diastereoisomeren Formen erhältlich.

Schmp. 83–84 °C (aus Tetrachlorkohlenstoff/Ether), $[\alpha]_D^{20}$ + 36,1 (c = 0,90, Methanol).

Schmp. 103–105 °C (aus Aceton/Ether), $[\alpha]_D^{20}$ + 42,8 (c = 0,841, Methanol).

Beispiel 2
2-(2-Hydroxy-3-tert.-butylamino-propyl)-isosorbid

Die Herstellung erfolgt analog Beispiel 1c, aus 2-(2,3-Epoxypropyl)-isosorbid und tert.-Butylamin. Schmp. 90–92 °C (aus Tetrachlorkohlenstoff), $[\alpha]_D^{20}$ + 170,8 (c = 0,612, Aceton).

Beispiel 3
2-(2-Hydroxy-3-tert.-butylamino-propyl)-isosorbid-5-nitrat

a) 2-Allyl-isosorbid-5-nitrat:

191 g Isosorbid-5-nitrat, 200 g Kaliumcarbonat und 150 ml Allylbromid werden in 300 ml Toluol 24 Stunden unter Rückfluß erhitzt. Nach dem Erkalten filtriert man von den anorganischen Salzen, wäscht mit etwas Toluol nach und extrahiert das Filtrat mehrmals mit Wasser. Die organische Phase wird i. Vak. eingeengt, wobei 160 g 2-Allyl-isosorbid-5-nitrat als öliges Rohprodukt zurückbleiben, das in dieser Form für die nachfolgende Umsetzung verwendet wird.

b) 2-(2,3-Epoxypropyl)-isosorbid-5-nitrat:

0,1 Mol rohes 2-Allyl-isosorbid-5-nitrat werden mit 25 g 3-Chlor-perbenzoesäure in 100 ml Chloroform 24 Stunden bei Raumtemperatur gerührt. Danach extrahiert man mit 2 N Natronlauge und engt die organische Phase ein. Das ölige Rohprodukt wird ohne weitere Reinigung in die Folgestufe eingesetzt.

c) 2-(2-Hydroxy-3-tert.-butylamino-propyl)-isosorbid-5-nitrat:

0,1 Mol rohes 2-(2,3-Epoxypropyl)-isosorbid-5-nitrat werden mit 0,3 Mol tert.-Butylamin und 10 ml Methanol 24 Stunden bei Raumtemperatur stehen gelassen. Danach engt man am Rotavapor i. Vak. ein. Der ölige Rückstand wird mit Fumarsäure in das Semifumarat überführt. Schmp. 167–168 °C (aus Ethanol), $[\alpha]_D^{20}$ + 72,3 (c = 0,775, $H_2O$).

Beispiel 4
5-(2-Hydroxy-3-tert.-butylamino-propyl)-isosorbid-2-nitrat

a) 5-Allyl-isosorbid-2-nitrat:

Aus 191 g Isosorbid-2-nitrat erhält man analog Beispiel 3a) 145 g öliges Rohprodukt, das in dieser Form weiter umgesetzt wird.

b) 5-(2,3-Epoxypropyl)-isosorbid-2-nitrat:

Analog Beispiel 3b) wird ein für die weitere Umsetzung genügend reines, öliges Produkt erhalten.

c) 5-(2-Hydroxy-3-tert.-butylamino-propyl)-isosorbid-2-nitrat:

Die Herstellung erfolgt analog Beispiel 3c). Maleat: Schmp. 118–120 °C (aus 2-Propanol), $[\alpha]_D^{20}$ + 37,6 (c = 0,732, $H_2O$).

Beispiel 5
5-(2-Hydroxy-3-tert.-butylamino-propyl)-isosorbid

a) 5-(2,3-Epoxypropyl)-isosorbid:

Durch Umsetzung von 5-Allyl-isosorbid mit 3-Chlor-perbenzoesäure analog Beispiel 1b) erhält man die Titelverbindung in Form eines farblosen Öls.

b) 5-(2-Hydroxy-3-tert.-butylamino-propyl)-isosorbid:

Maleat: Schmp. 139–142 °C (aus Ethanol), $[\alpha]_D^{20}$ + 35,8 (c = 0,838, $H_2O$).

Beispiel 6
5-(2-Hydroxy-3-isopropylamino-propyl)-isosorbid

Oxalat · 0,5 $H_2O$: Schmp. 54 °C (aus 2-Propanol), $[\alpha]_D^{20}$ + 38,9 (c = 0,757, $H_2O$).

Beispiel 7
2,5-Bis-(2-hydroxy-3-tert.-butylamino-propyl)-isosorbid

a) 2,5-Bis-(2,3-epoxypropyl)-isosorbid:

Herstellung nach US-Patent 3 272 845, Sdp.$_{0,1}$: 147 °C, $[\alpha]_D^{20}$ + 85,3 (c = 7,28, Methanol). Ein aus 2,5-Diallyl-isosorbid (Beispiel 1a) durch Umsetzung mit 3-Chlor-perbenzoesäure hergestelltes Produkt besitzt identische Eigenschaften.

b) 2,5-Bis-(2-hydroxy-3-tert.-butylamino-propyl)-isosorbid:

Oxalat: Schmp. 234–236 °C (aus Methanol/Aceton), $[\alpha]_D^{20}$ + 27,6 (c = 0,797, $H_2O$).

Beispiel 8
2,5-Bis-(2-hydroxy-3-isopropylamino-propyl)-isosorbid

Dioxalat: Schmp. 143–145 °C (aus Methanol/Aceton), $[\alpha]_D^{20}$ + 26,4 (c = 1,16, $H_2O$).

Beispiel 9
2-(2-Hydroxy-3-isopropylamino-propyl)-isosorbid-5-nitrat

Schmp. 76–78 °C (aus Ether), $[\alpha]_D^{20}$ + 44,2 (c = 0,775, $H_2O$).

Beispiel 10
2-(2-Hydroxy-3-isopropylamino-propyl)-isomannid

a) 2-Allyl-isomannid, 2,5-Diallyl-isomannid:

450 g Isomannid werden analog Beispiel 1a) mit Allylbromid umgesetzt und aufgearbeitet. Man erhält

80 g 2,5-Diallyl-isomannid, Sdp.$_{0,1}$: 94–100 °C
310 g 2-Allyl-isomannid, Sdp.$_{0,1}$: 85–90 °C

b) 2-(2,3-Epoxypropyl)-isomannid:

Die Herstellung erfolgt analog Beispiel 1b), durch Epoxydierung von 2-Allyl-isomannid mittels 3-Chlor-perbenzoesäure. Das erhaltene ölige Rohprodukt wird ohne weitere Reinigung in die nächste Stufe eingesetzt.

c) 2-(2-Hydroxy-3-isopropylamino-propyl)-isomannid:

Analog Beispiel 1c). Schmp. 80–82 °C (aus Ethylacetat) $[\alpha]_D^{20}$ + 101,4 (c = 0,764, $H_2O$).

Beispiel 11
2-(2-Hydroxy-3-isopropylamino-propyl)-isomannid-5-nitrat

a) 2-Allylisomannid-5-nitrat:

Zu einer Mischung aus 13 g 65-proz. Salpetersäure und 45 ml Essigsäureanhydrid tropft man unter Rühren, bei 0–5 °C 18,6 g 2-Allyl-isomannid. Nachdem man weitere 15 Min. bei dieser Temperatur belassen hat, gießt man in Wasser und extrahiert mit Dichlormethan. Die organische Phase wird am Rotationsverdampfer eingeengt. Als Rückstand verbleiben 23 g öliges 2-Allylisomannid-5-nitrat.

b) 2-(2,3-Epoxypropyl)-isomannid-5-nitrat:

Aus dem nach a) erhaltenen Rohprodukt durch Umsetzung mit 3-Chlor-perbenzoesäure, analog Beispiel 1b). Man erhält ein öliges Rohprodukt, das in dieser Form in die Folgestufe eingesetzt wird.

c) 2-(2-Hydroxy-3-isopropylamino-propyl)--isomannid-5-nitrat

Analog Beispiel 1c), aus obigem, rohen Epoxid und Isopropylamin.

Oxalat: Schmp. 81–82 °C (Aceton/Ether), $[\alpha]_D^{20}$ + 157 (c = 0,793, Methanol).

Beispiel 12
2,5-Bis-(2-hydroxy-3-isopropylamino-propyl)-isomannid
a) 2,5-Bis-(2,3-epoxypropyl)-isomannid:
Analog Beispiel 7a) aus 2,5-Diallyl-isomannid und 3-Chlor-perbenzoesäure. Das ölige Rohprodukt ist für die weitere Umsetzung geeignet.
b) 2,5-Bis-(2-hydroxy-3-isopropylamino-propyl)-isomannid:
Dioxalat · 0,5 $H_2O$: Schmp. 124–128 °C (aus Methanol), $[\alpha]_D^{20}$ + 64,3 (c = 0,848, $H_2O$).

Beispiel 13
2-(2-Hydroxy-3-isopropylamino-propyl)-isoidid
a) 2-Allyl-isoidid, 2,5-Diallyl-isoidid:
Gemäß Beispiel 1a) wird durch Umsetzung von Isoidid mit Allylbromid als Hauptprodukt 2-Allyl-isoidid erhalten, Sdp.$_{0,1}$: 110–111 °C. 2,5 Diallyl-isoidid entsteht nur in untergeordneter Menge.

b) 2-(2,3-Epoxypropyl)-isoidid:
Wird analog 1b) als öliges Rohprodukt erhalten und in dieser Form weiter umgesetzt.

c) 2-(2-Hydroxy-3-isopropylamino-propyl)-isoidid:
Oxalat · 0,5 $H_2O$: Schmp. 75–80 °C (aus Acetonitril), $[\alpha]_D^{20}$ + 18,05 (c = 0,637, $H_2O$).

Beispiel 14
2-(2-Hydroxy-3-isoprolyamino-propyl)-isoidid-5-nitrat
a) 2-Allyl-isoidid-5-nitrat:
Aus 2-Allyl-isoidid und Salpetersäure analog Beispiel 11a) erhält man ein öliges Rohprodukt, das ohne weitere Reinigung weiter umgesetzt wird.

b) 2-(2,3-Epoxypropyl)-isoidid-5-nitrat:
Analog 11b), öliges Rohprodukt

c) 2-(2-Hydroxy-3-isopropylamino-propyl)-isoidid-5-nitrat:
Oxalat: Schmp. 117–118 °C (aus Ethanol), $[\alpha]_D^{20}$ + 30,1 (c = 0,747, Methanol).

Beispiel 15
2-(2-Hydroxy-3-isopropylamino-propyl)-isosorbid-5-stearat
a) 2-Allyl-isosorbid-5-stearat:
Eine Mischung aus 18,6 g 2-Allyl-isosorbid, 29,8 g Stearinsäure-methylester und 0,1 g Natriummethylat werden im Wasserstrahlvakuum 2 Stunden auf 100 °C erhitzt. Anschließend destilliert man das Produkt i. Vak. Sdp.$_{0,05}$: 210 °C

b) 2-(2,3-Epoxypropyl)-isosorbid-5-stearat:
Durch Umsetzung vorstehender Verbindung mit m-Chlor-perbenzoesäure in Chloroform und übliche Aufarbeitung. Das Produkt wird in öliger Form erhalten und direkt weiter umgesetzt.

c) 2-(2-Hydroxy-3-isopropylamino-propyl)-isosorbid-5-stearat:
Reaktion des vorstehenden Epoxyds mit Isopropylamin liefert das gewünschte Produkt. Tartrat · 2 $H_2O$: Schmp. 58–60 °C (aus Ethylacetat), $[\alpha]_D^{20}$ + 32,9 (c = 0,849, $H_2O$).

Beispiel 16
2-(2-Hydroxy-3-isopropylamino-propyl)-isosorbid-5-isobutyrat
a) 2-Allyl-isosorbid-5-isobutyrat:
18,6 g 2-Allyl-isosorbid werden mit 15,8 g Isobuttersäureanhydrid unter Zusatz von 0,1 g p-Toluol-sulfonsäure 2 Stunden bei 60 °C gerührt. Danach destilliert man i. Vak. Sdp.$_{0,05}$: 107 °C

b) 2-(2,3-Epoxypropyl)-isosorbid-5-isobutyrat:
Wird als öliges Rohprodukt erhalten und in dieser Form weiter verwendet.

c) 2-(2-Hydroxy-3-isopropylamino-propyl)-isosorbid-5-isobutyrat:
Oxalat · 0,25 $H_2O$: Schmp. 100 °C (aus Ethanol), $[\alpha]_D^{20}$ + 53,4 (c = 0,861, $H_2O$).

Beispiel 17
2-(2-Hydroxy-3-isopropylamino-propyl)-isosorbid-5-pivaloat
a) 2-Allyl-isosorbid-5-pivaloat:
18,6 g 2-Allyl-isosorbid, 12,0 g Pivaloylchlorid und 25 ml Pyridin werden in 100 ml Dichlormethan 30 Min. bei Raumtemperatur gerührt. Danach extrahiert man mit Wasser und engt die Methylenchloridphase ein. Der Rückstand wird i. Vak. destilliert. Sdp.$_{0,05}$: 106 °C.

b) 2-(2,3-Epoxypropyl)-isosorbid-5-pivaloat:
Wird als öliges Rohprodukt erhalten und in dieser Form weiter verwendet.

c) 2-(2-Hydroxy-3-isopropylamino-propyl)-isosorbid-5-pivaloat:
Oxalat: Schmp. 119–121 °C (aus Aceton), $[\alpha]_D^{20}$ + 50,3 (c = 0,815, $H_2O$).

Beispiel 18
2-(2-Hydroxy-3-isopropylamino-propyl)-isosorbid-5-caprinat
a) 2-(2,3-Epoxypropyl)-isosorbid-5-caprinat:
Zu einer Lösung von 20,2 g 2-(2,3-Epoxypropyl)-isosorbid (Isomer vom Schmp. 88–90 °C, siehe Beispiel 1) und 10 g Pyridin in 50 ml Dichlormethan tropft man bei 0–5 °C eine Lösung von 19,0 g Caprinsäurechlorid in 50 ml Dichlormethan. Nachdem man noch 2 Stunden bei Raumtemperatur gerührt hat, versetzt man mit Wasser, trennt die Schichten und engt die organische Phase ein. Der zurückbleibende, ölige Rückstand wird in dieser Form weiter umgesetzt.

b) 2-(2-Hydroxy-3-isopropylamino-propyl)-isosorbid-5-caprinat:
Oxalat: Schmp. 113–115 °C (aus Aceton), $[\alpha]_D^{20}$ + 30,6 (c = 1,177, $H_2O$).

Beispiel 19
2-(2-Hydroxy-3-isopropylamino-propyl)-isosorbid-5-acetat
a) 2-(2,3-Epoxypropyl)-isosorbid-5-acetat:
Analog Beispiel 18a) aus 2-(2,3-Epoxypropyl)-isosorbid (Isomer vom Schmp. 88–90 °C) und Acetylchlorid. $Sdp._{0,1}$: 128 °C.

b) 2-(2-Hydroxy-3-isopropylamino-propyl)-isosorbid-5-acetat:
Oxalat: Schmp. 113–114 °C (aus Ethanol, $[\alpha]_D^{20}$ + 50,6 (c = 1,018, Methanol).

Beispiel 20
2-(2-Hydroxy-3-isopropylamino-propyl)-isosorbid-5-undecylenat
a) 2-(2,3-Epoxypropyl)-isosorbid-5-undecylenat:
Analog Beispiel 18a) aus 2-(2,3-Epoxypropyl)-isosorbid (Isomer vom Schmp. 88–90 °C) und Undecylensäurechlorid. Öliges Rohprodukt.

b) 2-(2-Hydroxy-3-isopropylamino-propyl)-isosorbid-5-undecylenat:
Oxalat: Schmp. 111 °C (aus Aceton), $[\alpha]_D^{20}$ + 37,3 (c = 1,355, Methanol).

Beispiel 21
2-[2-Hydroxy-3-(7-theophyllinyl)-propyl]-isosorbid
10,1 g 2-(2,3-Epoxypropyl)-isosorbid werden mit 9,0 g Theophyllin in 150 ml 1-Propanol, unter Zusatz von 1 ml Pyridin, 3 Stunden am Rückfluß gekocht. Danach engt man i. Vak. ein und löst den Rückstand in Wasser. Man alkalisiert mit verdünnter wäßriger Natriumhydroxid-Lösung, sättigt mit Natriumchlorid und extrahiert 5mal mit je 100 ml Dichlormethan. Die vereinigten organischen Extrakte werden über $Na_2SO_4$ getrocknet und i. Vak. eingeengt, der Rückstand aus Ethanol umkristallisiert.
Ausbeute: 12,4 g, Schmp. 155–156 °C, $[\alpha]_D^{20}$ + 2,86 (c = 0,699, $H_2O$).

Beispiel 22
2-[2-Hydroxy-3-(7-theophyllinyl)-propyl]-isosorbid-5-nitrat
Aus 2-(2,3-Epoxypropyl)-isosorbid-5-nitrat und Theophyllin analog vorstehendem Beispiel. Schmp. 145–146 °C (aus Ethanol), $[\alpha]_D^{20}$ + 91,2 (c = 0,822, Aceton).

Beispiel 23
5-[2-Hydroxy-3-(7-theophyllinyl)-propyl]-isosorbid-2-nitrat
Aus 5-(2,3-Epoxypropyl)-isosorbid-2-nitrat und Theophyllin. Schmp. 143–144 °C (aus 2-Propanol), $[\alpha]_D^{20}$ + 39,3 (c = 0,788, Aceton).

Beispiel 24
Bis-2,5[2-hydroxy-3-(7-theophyllinyl)-propyl]-isosorbid
Aus 2,5-Bis-(2,3-epoxypropyl)-isosorbid und Theophyllin. Die Substanz wurde als farbloses, amorphes Pulver erhalten, das 0,25 Mol $H_2O$ enthält. Schmp. 155–160 °C (Zers.) (aus 2-Propanol), $[\alpha]_D^{20}$ + 217,6 (c = 0,594, Aceton).

Beispiel 25
2-[2-Hydroxy-3-(1-theobrominyl)-propyl]-isosorbid
Aus 2-(2,3-Epoxypropyl)-isosorbid und Theobromin. Schmp. 125–127 °C (aus 2-Propanol), $[\alpha]_D^{20}$ + 27,0 (c = 0,629, Methanol).

Beispiel 26
5-[3-[N-Benzyl-N-(2-(7-theophyllinyl)-ethylamino)]-2-hydroxy-propyl]-isosorbid
0,1 Mol 7-(2-Benzylamino-ethyl)-theophyllin (DAS 1 011 424) und 0,1 Mol 5-(2,3-Epoxypropyl)-isosorbid werden in 300 ml Ethanol 20 Stunden unter Rückfluß erhitzt. Beim Abkühlen auf 0 °C kristallisiert das Reaktionsprodukt aus. Man saugt ab und kristallisiert aus Methanol um. Ausbeute 68% d. Th. Schmp. 150–151 °C, $[\alpha]_D^{20}$ + 38,5 (c = 0,818, Methanol).

Beispiel 27
2-[3-[N-Benzyl-N-(2-(7-theophyllinyl)-ethylamino)]-2-hydroxy-propyl]-isosorbid
Aus 7-(2-Benzylamino-ethyl)-theophyllin und 2-(2,3-Epoxypropyl)-isosorbid analog vorstehendem Beispiel. Hydrochlorid · 0,5 $H_2O$: sintert ab 88–94 °C (aus Aceton), $[\alpha]_D^{20}$ + 14,8 (c = 0,88, Methanol).

Beispiel 28
5-[3-[2-(7-Theophyllinyl)-ethylamino]-2-hydroxy-propyl]-isosorbid
10,3 g 5-[3-[N-Benzyl-N-(2-(7-theophyllinyl)-ethylamino)]-2-hydroxy-propyl]-isosorbid (Beispiel 26) werden in 100 ml 80-proz. Methanol gelöst, mit 2 g 5-proz. Pd/C versetzt und bei Raumtemperatur hydriert bis die Wasserstoffaufnahme beendet ist. Man filtriert vom Katalysator ab und engt das Filtrat ein. Der Rückstand wird in Methanol gelöst und mit etherischer HCl das Hydrochlorid gefällt. Nach dem Umkristallisieren aus 2-Propanol erhält man das gewünschte Produkt als Hydrochlorid in 79% Ausbeute in Form farbloser Kristalle, die ab 82–86 °C zu sintern beginnen. $[\alpha]_D^{20}$ + 32,3 (c = 0,743, Methanol).

Beispiel 29
5-[3-[N-Benzyl-N-(2-(7-theophyllinyl)-ethylamino)]-2-hydroxy-propyl]-isosorbid-2-nitrat
Aus 7-(2-Benzylamino-ethyl)-theophyllin und 5-(2,3-Epoxypropyl)-isosorbid-2-nitrat. Hydrochlorid · 0,25 $H_2O$: sintert ab 82–87 °C (aus 2-Propanol), $[\alpha]_D^{20}$ + 32,8 (c = 0,883, Methanol).

Beispiel 30
2-[3-[N-Benzyl-N-(3-(7-theophyllinyl)-propylamino)]-2-hydroxy-propyl]-isosorbid
Aus 7-(3-Benzylamino-propyl)-theophyllin [Chem. Ber. 90, 1651 (1957)] und 2-(2,3-Epoxypropyl)-isosorbid. Hydrochlorid · 0,5 $H_2O$: sintert ab 85–90 °C (aus 2-Propanol), $[\alpha]_D^{20}$ + 15,5 (c = 1,097, Methanol).

Beispiel 31
2-[3-[3-(7-Theophyllinyl)-propylamino]-2-hydroxy-propyl]-isosorbid

Aus 2-[3-[N-Benzyl-N-(3-(7-theophyllinyl)-propylamino)]-2-hydroxy-propyl]-isosorbid (Beispiel 30) durch katalytische Hydrierung mit Pd/C. Hydrochlorid: sintert ab 162–165 °C (aus Ethanol, $[\alpha]_D^{20}$ + 13,1 (c = 0,648, Methanol).

Beispiel 32
2-[3-[2-(7-Theophyllinyl)-ethylamino]-2-hydroxy-propyl]-isosorbid

Aus 2-[3-[N-Benzyl-N-(2-(7-theophyllinyl)-ethylamino)]-2-hydroxy-propyl]-isosorbid (Beispiel 27) durch katalytische Hydrierung. Hydrochlorid · 0,5 $H_2O$: sintert ab 98–100 °C (aus Ethanol), $[\alpha]_D^{20}$ + 20,3 (c = 0,885, Methanol).

Beispiel 33
2-[3-[N-Benzyl-N-(2-(7-theophyllinyl)-propylamino)]-2-hydroxy-propyl]-isosorbid-5-nitrat

Aus 7-(3-Benzylamino-propyl)-theophyllin und 2-(2,3-Epoxypropyl)-isosorbid-5-nitrat. Hydrochlorid · 0,5 $H_2O$: sintert ab 98–102 °C (aus 2-Propanol), $[\alpha]_D^{20}$ + 54,4 (c = 1,076, Methanol).

Beispiel 34
2-[3-[N-Benzyl-N-(3-(7-theophyllinyl)-propyl)amino]-2-hydroxy-propyl]-isomannid

Aus 7-(3-Benzylamino-propyl)-theophyllin und 2-(2,3-Epoxypropyl)-isomannid. Hydrochlorid · $H_2O$: sintert ab 94–97 °C (aus 2-Propanol), $[\alpha]_D^{20}$ + 42,6 (c = 0,716, Methanol).

Beispiel 35
2-[3-[3-(7-Theophyllinyl)-propylamino]-2-hydroxy-propyl]-isomannid

Aus der in Beispiel 34 beschriebenen Verbindung durch katalytische Hydrierung. Hydrochlorid · 0,75 $H_2O$: sintert ab 143–153 °C (aus 2-Propanol), $[\alpha]_D^{20}$ + 54,9 (c = 0,784, Methanol).

Beispiel 36
5-[3-[2-(7-Theophyllinyl)-ethylamino]-2-hydroxy-propyl]-isosorbid-2-nitrat

Aus 7-(2-Amino-ethyl)-theophyllin [DAS 1 011 424] und 5-(2,3-Epoxypropyl)-isosorbid-2-nitrat. Hydrochlorid · 0,25 $H_2O$: sintert ab 76–80 °C (aus 2-Propanol), $[\alpha]_D^{20}$ + 37,0 (c = 0,81, Methanol).

Beispiel 37
2-[3-[3-(7-Theophyllinyl)-propylamino]-2-hydroxy-propyl]-isosorbid-5-nitrat

Aus 7-(3-Amino-propyl)-theophyllin [Collect. Czech. Chem. Commun. 38, 1571 (1973)] und 2-(2,3-Epoxypropyl)-isosorbid-5-nitrat. Hydrochlorid · 0,5 $H_2O$: sintert ab 83–86 °C (aus 2-Propanol), $[\alpha]_D^{20}$ + 63,1 (c = 0,919, Methanol).

Beispiel 38
2-[3-($N^6$-Benzoyl-9-adenyl)-2-hydroxy-propyl]-isosorbid

Aus $N^6$-Benzoyl-adenin und 2-(2,3-Epoxypropyl)-isosorbid. Schmp. 168–169 °C (aus Ethanol/Ethylacetat), $[\alpha]_D^{20}$ + 20,0 (c = 0,801, Methanol).

Beispiel 39
2-(3-(9-Adenyl)-2-hydroxy-propyl]-isosorbid

Aus der in Beispiel 38 beschriebenen Verbindung durch Abspaltung der Benzoylgruppe mittels Natriummethylat in methanolischer Lösung. Die Substanz kristallisiert aus Ethanol mit 0,25 Mol $H_2O$. Schmp. 172–175 °C, $[\alpha]_D^{20}$ + 24,0 (c = 0,811, $H_2O$).

Beispiel 40
Bis-2,5-[3-[N-benzyl-N-(2-(7-theophyllinyl)-ethyl)-amino]-2-hydroxy-propyl]-isomannid

Aus 7-(2-Benzylamino-ethyl)-theophyllin und 2,5-Bis-(2,3-epoxypropyl)-isomannid. Dihydrochlorid · 0,5 $H_2O$: sintert ab 146–148 °C (aus 2-Propanol), $[\alpha]_D^{20}$ + 44,7 (c = 0,795, Methanol).

Beispiel 41
Bis-2,5-[3-[2-(7-theophyllinyl)-ethylamino]-2-hydroxy-propyl]-isommanid

Aus vorstehender Verbindung (Beispiel 40) durch katalytische Hydrierung. Dihydrochlorid · $H_2O$: sintert ab 127–132 °C (aus Ethanol), $[\alpha]_D^{20}$ + 51,8 (c = 0,733, Methanol).

Beispiel 42
2-[3-(9-(6-Methylmercaptopurinyl))-2-hydroxy-propyl]-isosorbid

Aus 2-(2,3-Epoxypropyl)-isosorbid und 6-Methylmercaptopurin. Sintert ab 55–60 °C (aus Toluol), $[\alpha]_D^{20}$ + 12,8 (c = 0,98, Methanol).

**Patentansprüche**

1. 1,4 : 3,6-Dianhydro-hexit-Derivate der allgemeinen Formel I,

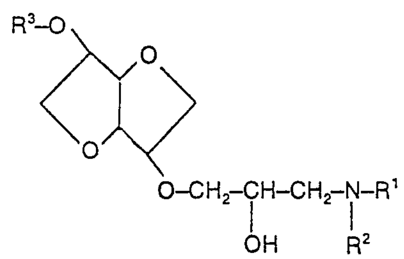

I

worin

$R^1$ Wasserstoff oder eine Benzylgruppe bedeutet,

$R^2$ einen geradkettigen oder verzweigten Alkylrest mit 1 bis 4 Kohlenstoffatomen oder einen ω-Theophyllin-7-yl-alkyl-Rest, wobei dieser Alkylrest 2 bis 3 Kohlenstoffatome aufweisen kann, bedeutet

oder worin

$R^1$ und $R^2$ gemeinsam mit dem Stickstoffatom, an das sie gebunden sind, den Theophyllin-7-yl-Rest, den Theobromin-1-yl-Rest, den an der 6-Aminogruppe gegebenenfalls mono- oder disubstituierten Aden-9-yl-Rest, den an der 2-Aminogruppe gegebenenfalls substituierten Guan-9-yl-Rest, den 6-Chlor-purin-9-yl-Rest oder den 6-Alkylmercaptopurin-9-yl-Rest oder den an der Aminogruppe gegebenenfalls substituierten 2-Amino-6-Chlor-purin-9-yl-Rest
und worin
$R^3$ Wasserstoff, einen geradkettigen oder verzweigten, gesättigten oder ungesättigten Acylrest mit 1 bis 20 Kohlenstoffatomen, einen Pyridylcarbonylrest, eine $NO_2$-Gruppe oder einen Rest der Formel

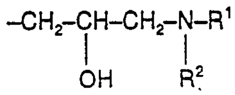

darstellt, worin $R^1$ und $R^2$ die oben angegebene Bedeutung besitzen,
sowie deren Salze mit anorganischen und organischen Säuren.

2. 1,4 : 3,6-Dianhydro-hexit-Derivate gemäß Anspruch 1, dadurch gekennzeichnet, daß sie Derivate des 1,4 : 3,6-Dianhydro-L-idits (Isoidid) der allgemeinen Formel II sind,

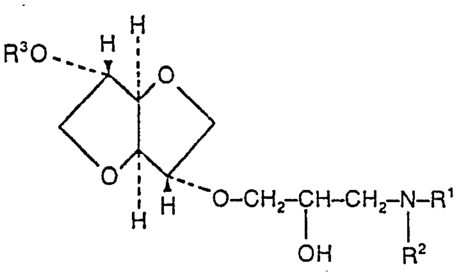

II

in welcher $R^1$, $R^2$ und $R^3$ die in Anspruch 1 angegebene Bedeutung besitzen, sowie deren Salze.

3. 1,4 : 3,6-Dianhydro-hexit-Derivate gemäß Anspruch 1, dadurch gekennzeichnet, daß sie Derivate des 1,4 : 3,6-Dianhydro-D-glucits (Isosorbid) der allgemeinen Formeln III und IV sind,

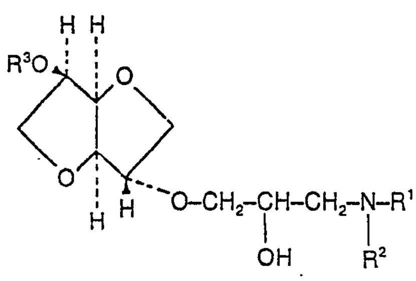

III

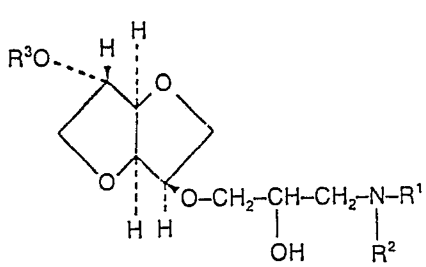

IV

in welchen $R^1$, $R^2$ und $R^3$ die in Anspruch 1 angegebene Bedeutung besitzen, sowie deren Salze.

4. 1,4 : 3,6-Dianhydro-hexit-Derivate gemäß Anspruch 1, dadurch gekennzeichnet, daß sie Derivate des 1,4 : 3,6-Dianhydro-D-mannits (Isomannid) der allgemeinen Formel V sind

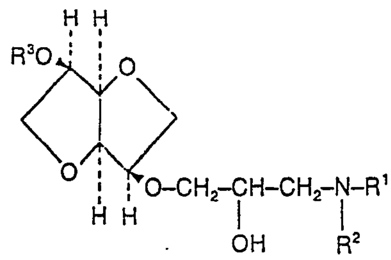

V

in welcher $R^1$, $R^2$ und $R^3$ die in Anspruch 1 angegebene Bedeutung besitzen, sowie deren Salze.

5. Verfahren zur Herstellung der 1,4 : 3,6-Dianhydro-hexit-Derviate gemäß Anspruch 1, dadurch gekennzeichnet, daß man einen (2,3-Epoxypropyl)-1,4 : 3,6-dianhydro-hexit der allgemeinen Formel VI

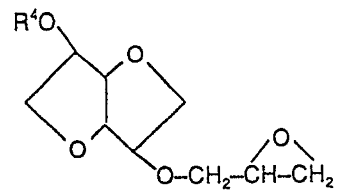

VI

worin $R^4$ Wasserstoff, einen geradkettigen oder verzweigten, gesättigten oder ungesättigten Acylrest mit 1 bis 20 Kohlenstoffatomen, einen Pyridylcarbonylrest, eine $NO_2$-Gruppe oder einen 2,3-Epoxypropylrest der Formel

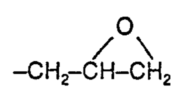

darstellt, in an sich bekannter Weise mit einer Verbindung der allgemeinen Formel VII,

$$HNR^1R^2 \qquad VII$$

worin $R^1$ und $R^2$ die in Anspruch 1 angegebene Bedeutung besitzen, umsetzt und das Reaktionsprodukt der allgemeinen Formel I gegebenenfalls in ein Salz mit einer anorganischen oder organischen Säure überführt.

6. Verwendung der 1,4 : 3,6-Dianhydro-hexit-Derivate gemäß Anspruch 1 zur Herstellung von Arzneimitteln zur Bekämpfung und Prophylaxe von Herz- und Kreislauferkrankungen.

7. Arzneimittel enthaltend mindestens eine Verbindung der allgemeinen Formel I gemäß Anspruch 1 und gegebenenfalls übliche Hilfs- und Trägerstoffe.

**Claims**

1. 1,4 : 3,6-dianhydrohexite derivatives having the general Formula I,

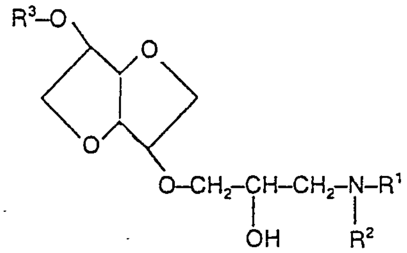

I

wherein

$R^1$ is hydrogen or a benzyl group,

$R^2$ is a straight-chain or branched-chain alkyl group having 1 to 4 carbon atoms or an omega-theophyllin-7-yl-alkyl group wherein the alkyl radical can have 2 to 3 carbon atoms

or wherein

$R^1$ and $R^2$ together with the nitrogen atom to which they are bound form the theophyllin-7-yl-group, the theobromine-1-yl-group, the adene-9-yl-group, optionally mono- or disubstituted at the 6-amino-group, the guan-9-yl-group, optionally substituted at the 2-amino-group, the 6-chloropurine-9-yl-group or the 6-allylmercaptopurine-9-yl-group or the 2-amino-6-chloro-purine-9-yl-group, optionally substituted at the amino-group

and wherein

$R^3$ is hydrogen, a straight-chain or branched-chain, saturated or unsaturated acyl group having 1 to 20 carbon atoms, a pyridylcarbonyl group, an $NO_2$-group or a group having the Formula

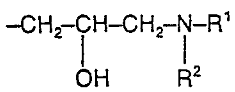

wherein $R^1$ and $R^2$ are as hereinbefore defined; and salts thereof with inorganic or organic acids.

2. 1,4 : 3,6-dianhydrohexite derivatives according to claim 1, characterized in that they are derivatives of 1,4 : 3,6-dianhydro-L-idite (isoidide) having the general Formula II

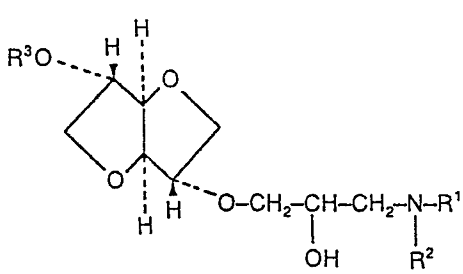

II

where $R^1$, $R^2$ and $R^3$ are as defined in claim 1; and salts thereof.

3. 1,4 : 3,6-dianhydrohexite derivatives according to claim 1, characterized in that they are derivatives of 1,4 : 3,6-dianhydro-D-glucites (isosorbides) having the general Formula III and IV

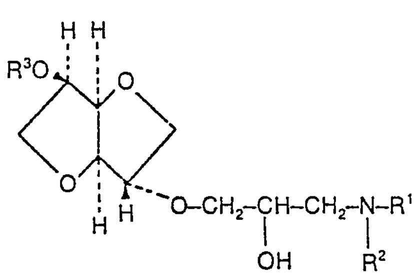

III

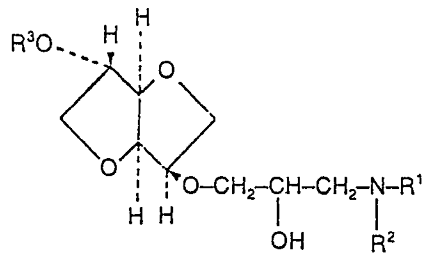

IV

in which $R^1$, $R^2$ and $R^3$ are as defined in claim 1; and salts thereof.

4. 1,4 : 3,6-dianhydrohexite derivatives according to claim 1, characterized in that they are derivatives of 1,4 : 3,6-dianhydro-D-mannite (isomannide) having the general Formula V

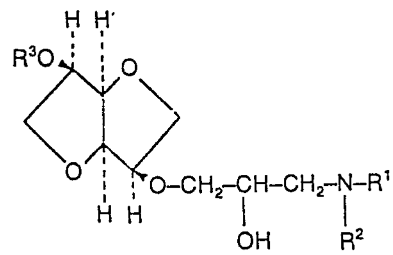

V

in which $R^1$, $R^2$ and $R^3$ are as defined in claim 1; and salts thereof.

5. Process for the preparation of 1,4 : 3,6-dianhydrohexite derivatives according to claim 1, characterized in that a (2,3-epoxypropyl)-1,4 : 3,6-dianhydrohexite having the general Formula VI

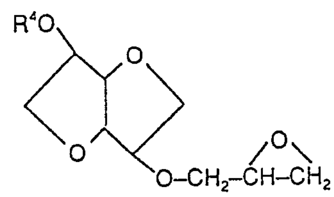

VI

wherein $R^4$ is hydrogen, a straight-chain or branched-chain, saturated or unsaturated acyl group having 1 to 20 carbon atoms, a pyridylcarbonyl group, an $NO_2$-group or a 2,3-epoxypropyl-group having the Formula

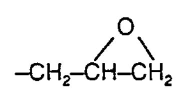

wherein $R^1$ and $R^2$ are as defined in claim 1 is reacted in a per se known manner with a compound having the general Formula VII

$$HNR^1R^2 \qquad VII$$

wherein $R^1$ and $R^2$ are as defined in claim 1; and, optionally, the reaction product having the general Formula I is converted to a salt of an inorganic or an organic acid.

6. Use of 1,4 : 3,6-dianhydrohexite derivatives according to claim 1 for the preparation of pharmaceuticals for combatting and prophylaxis of heart and circulatory deseases.

7. Pharmaceuticals containing at least one compound of general Formula I according to claim 1 and, optionally, ordinary auxiliary and carrier materials.

**Revendications**

1. Dérivés de 1,4 : 3,6-dianhydro-hexitols de formule générale I

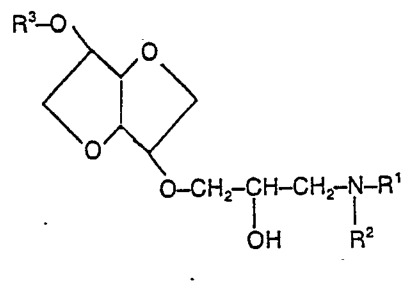

I

dans laquelle

$R^1$ désigne l'hydrogène ou un groupe benzyle,

$R^2$ est un reste alkyle à chaîne droite ou à chaîne ramifiée ayant 1 à 4 atomes de carbone ou un reste ω-théophylline-7-ylalkyle, ce reste alkyle pouvant présenter 2 ou 3 atomes de carbone,

oud dans laquelle

$R^1$ et $R^2$ forment conjointement avec l'atome d'azote auquel ils sont liés le reste théophylline-7-yle, le reste théobromine-1-yle, le reste adén-9-yle éventuellement monosubstitué ou disubstitué sur le groupe 6-amino, le reste guan-9-yle éventuellement substitué sur le groupe 2-amino, le reste 6-chloropurine-9-yle ou un reste 6-alkylmercaptopurine-9-yle ou le reste 2-amino-6-chloropurine-9-yle éventuellement substitué sur le groupe amino,

et dans laquelle

$R^3$ représente l'hydrogène, un reste acyle saturé ou non saturé, à chaîne droite ou ramifiée, ayant 1 à 20 atomes de carbone, un reste pyridylcarbonyle, un groupe $NO_2$– ou un reste de formule

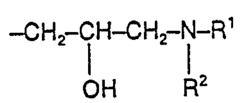

dans laquelle $R^1$ et $R^2$ ont la définition indiquée ci-dessus ainsi que leurs sels d'acides inorganiques et organiques.

2. Dérivés de 1,4 : 3,6-dianhydro-hexitols suivant la revendication 1, caractérisés en ce qu'ils sont des dérivés du 1,4 : 3,6-dianhydro-L-iditol (isoidide) de formule générale II

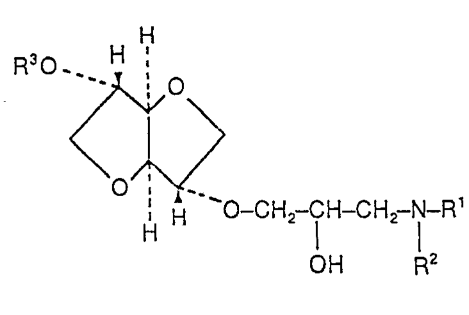

II

dans laquelle $R^1$, $R^2$ et $R^3$ ont la définition indiquée dans la revendication 1, ainsi que leurs sels.

3. Dérivés de 1,4 : 3,6-dianhydro-hexitols suivant la revendication 1, caractérisés en ce qu'ils sont des dérivés du 1,4 : 3,6-dianhydro-D-glucitol (isosorbide) de formules générales III et IV

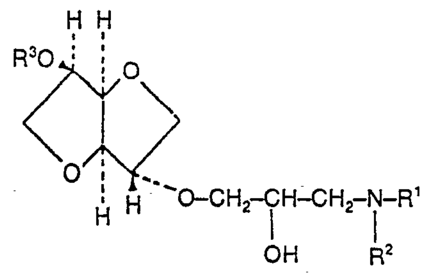

III

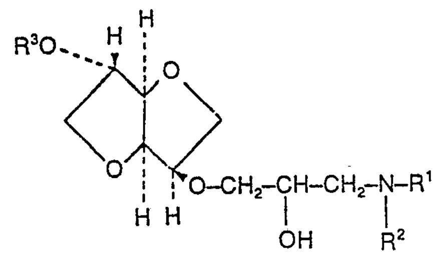

IV

dans lesquelles $R^1$, $R^2$ et $R^3$ ont la définition indiquée dans la revendication 1, ainsi que leurs sels.

4. Dérivés de 1,4 : 3,6-Dianhydro-hexitols suivant la revendication 1, caractérisés en ce qu'ils sont des dérivés du 1,4 : 3,6-dianhydro-D-mannitol (isomannide) de formule générale V

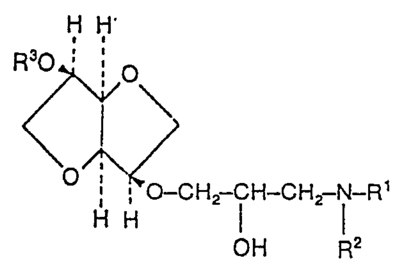

V

dans laquelle $R^1$, $R^2$ et $R^3$ ont la définition indiquée dans la revendication 1, ainsi que leurs sels.

5. Procédé de production des dérivés de 1,4 : 3,6-dianhydro-hexitols suivant la revendication 1, caractérisé en ce qu'on prépare un (2,3-époxypropyl)-1,4 : 3,6-dianhydro-hexitol de formule générale VI

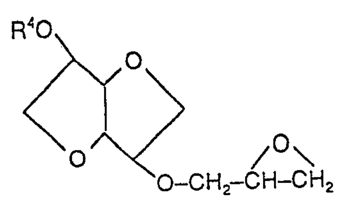

VI

dans laquelle $R^4$ est l'hydrogène, un reste acyle saturé ou non saturé à chaîne droite ou ramifiée ayant 1 à 20 atomes de carbone, un reste pyridylcarbonyle, un groupe $NO_2$ ou un reste 2,3-époxypropyle de formule

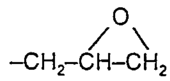

on le fait réagir d'une manière connue avec un composé de formule générale VII

$$HNR^1R^2 \qquad VII$$

dans laquelle $R^1$ et $R^2$ ont la définition indiquée dans la revendication 1 et on transforme le produit de réaction de formule générale I le cas échéant en un sel avec un acide inorganique ou organique.

6. Utilisation des dérivés de 1,4 : 3,6-dianhydrohexitols suivant la revendication 1 pour la préparation de médicaments destinés à la lutte curative et préventive contre des maladies cardiaques et des maladies du système circulatoire.

7. Médicament contenant au moins un composé de formule générale I suivant la revendication 1 et, le cas échéant, des adjuvants et des supports classiques.